# DEMANDE DE BREVET EUROPEEN

(11) **EP 4 435 082 A1**
(43) Date de publication de la demande: **25.09.2024**
(21) Numéro de dépôt: 24164813.8
(22) Date de dépôt: 20.03.2024
(51) Int. Cl.: C12M 1/107, C12M 1/16, C12M 1/00

(54) **INSTALLATION POUR LA MÉTHANISATION DE MATIÈRE ORGANIQUE**

(30) Priorité: 20.03.2023 FR 2302573
(71) Demandeur: Octometha, 60200 Compiègne (FR)
(72) Inventeur: BACHET, Vincent, 77190 DAMMARIE LES LYS (FR)
(74) Mandataire: Lavoix

(57) **Abrégé**

La présente invention concerne une installation (10) de méthanisation, comprenant : un couloir (12) de digestion, apte à recevoir de la matière organique,
- un collecteur (14) de gaz, surmontant le couloir de digestion ; et
- un dispositif d'introduction (16) et un dispositif d'évacuation (18) de matière organique, disposés aux extrémités du couloir de digestion ;

le couloir de digestion comprenant une surface sensiblement cylindrique s'étendant selon la direction longitudinale ;
l'installation étant caractérisée en ce que le couloir de digestion comporte en outre : une zone d'introduction (32), présentant une largeur croissante de manière continue ; et une zone d'évacuation (34), présentant une largeur décroissante de manière continue.

## Description

La présente invention concerne une installation de méthanisation, du type comportant : un couloir de digestion, apte à recevoir de la matière organique, ledit couloir s'étendant de manière sensiblement linéaire selon une direction longitudinale horizontale, entre une première et une seconde extrémités ; un collecteur de gaz, surmontant le couloir de digestion ; et un dispositif d'introduction et un dispositif d'évacuation de matière organique, disposés respectivement à la première et à la seconde extrémités du couloir de digestion ; le couloir de digestion comprenant une zone principale, ladite zone principale présentant une surface sensiblement cylindrique s'étendant selon la direction longitudinale.

La méthanisation permet notamment de valoriser de la matière organique issue de l'agriculture, de type lisier ou fumier. Par un processus de fermentation, ou digestion, ladite matière organique conduit à du biogaz, ou gaz de digestion, et à un résidu solide, ou digestat.

La présente invention concerne plus particulièrement la méthanisation dite « par voie sèche ». Dans ce type de méthanisation, la matière organique comprend une certaine quantité de matière sèche, qui la rend difficile ou impossible à pomper par une pompe à rotor.

En particulier, lorsque la matière organique comprend à la fois une certaine quantité d'eau et une certaine quantité de paille, ladite matière organique présente des propriétés physiques d'un solide en son centre et des propriétés physiques d'un liquide à sa périphérie. Il est ainsi possible de provoquer un déplacement linéaire de ladite matière organique, respectivement en l'alimentant et en l'évacuant à chacune des extrémités du trajet.

Une installation de méthanisation, réalisée sur ce principe, est connue du document FR3107637 au nom du Demandeur.

Une telle installation de méthanisation n'offre cependant pas de résultat optimal en termes de déplacement de la matière organique entre l'entrée et la sortie.

L'invention a pour objet d'améliorer l'installation existante. A cet effet, l'invention a pour objet une installation de méthanisation du type précité, dans laquelle le couloir de digestion comporte en outre : une zone d'introduction, présentant une largeur croissante de manière continue entre la première extrémité et la zone principale ; et une zone d'évacuation, présentant une largeur décroissante de manière continue entre la zone principale et la seconde extrémité.

Suivant d'autres aspects avantageux de l'invention, l'installation comporte l'une ou plusieurs des caractéristiques suivantes, prises isolément ou suivant toutes les combinaisons techniquement possibles :
- selon une direction transversale, perpendiculaire à la direction longitudinale, au moins l'une de la zone d'introduction et de la zone d'évacuation est délimitée par deux premières parois sensiblement planes ;
- chacune des deux premières parois est sensiblement verticale ;
- chacune des premières parois forme un premier angle compris entre 30° et 60°, préférentiellement compris entre 35° et 55°, plus préférentiellement compris entre 40° et 50°, avec la direction longitudinale ;
- le couloir de digestion comporte deux deuxièmes parois orientées vers le haut, chacune desdites deuxièmes parois étant inclinée par rapport à la verticale, d'un deuxième angle compris entre 30° et 60°, préférentiellement compris entre 40° et 50° ;
- le couloir de digestion comporte en outre un fond sensiblement horizontal, reliant les deux deuxièmes parois ;
- la zone principale du couloir de digestion comporte deux troisièmes parois sensiblement verticales, chacune desdites troisièmes parois prolongeant vers le haut l'une des deuxièmes parois ;
- le dispositif d'introduction comporte : un conduit d'introduction, ouvrant sur la première extrémité du couloir de digestion ; et un poussoir, apte à pousser la matière organique vers ladite première extrémité ;
- le conduit d'introduction comporte : une portion de chute, s'étendant verticalement ; et une portion de déflexion, configurée pour introduire la matière organique dans le couloir de digestion selon un déplacement sensiblement horizontal ; le poussoir étant apte à se déplacer selon une course verticale dans la portion de chute ;
- la portion de déflexion comporte une glissière inclinée vers la première extrémité du couloir de digestion.

L'invention se rapporte en outre à un procédé de construction d'une installation telle que décrite ci-dessus, comprenant les étapes suivantes : réalisation d'une excavation dans un sol ; et application d'une membrane sur ladite excavation, de sorte à former les deuxièmes parois.

L'invention apparaîtra plus clairement à la lecture de la description qui va suivre, donnée uniquement à titre d'exemple non limitatif, et faite en référence aux dessins dans lesquels :
[Fig. 1] la figure 1 est une vue en coupe longitudinale d'une installation de méthanisation selon un mode de réalisation de l'invention ;
[Fig. 2] la figure 2 est une vue en coupe horizontale de l'installation de la figure 1 ; et
[Fig. 3] la figure 3 est une vue en section transversale de l'installation des figures 1 et 2.

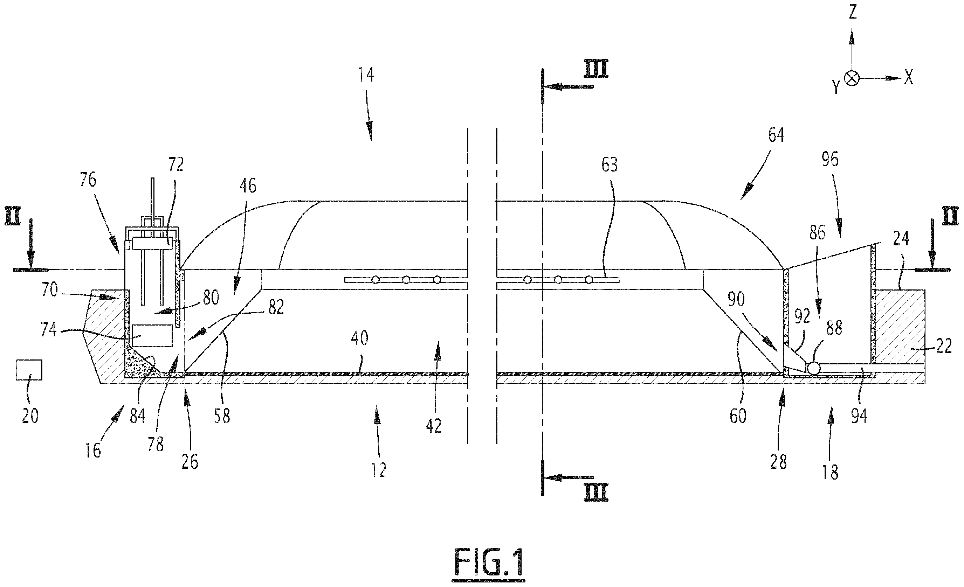

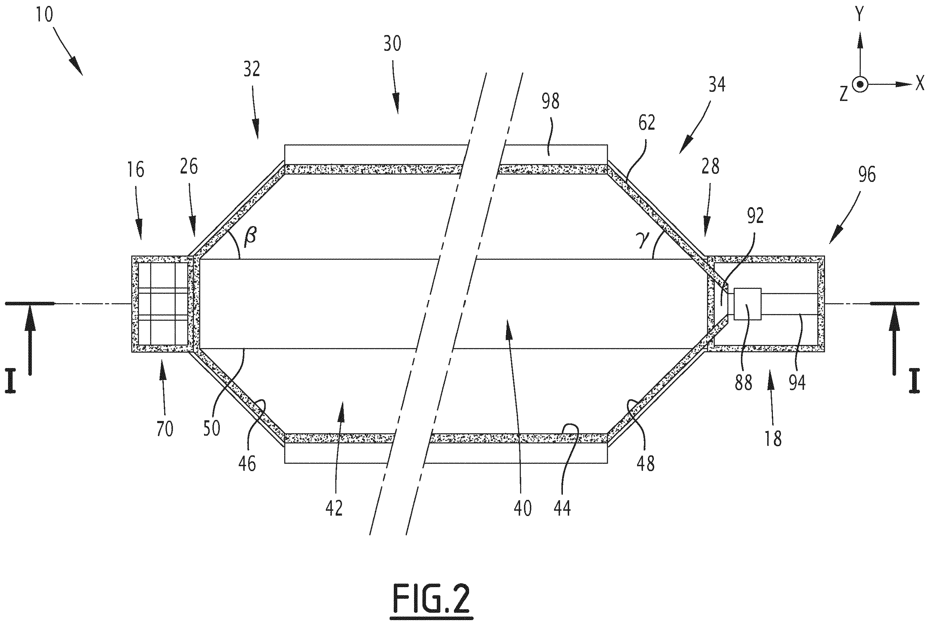

Les figures 1 à 3 représentent une installation 10 de méthanisation, selon un mode de réalisation de l'invention.

L'installation 10 comprend : un couloir 12 de digestion ; un collecteur 14 de gaz ; un dispositif 16 d'introduction ; un dispositif 18 d'évacuation ; et un module électronique 20 de commande, représenté schématiquement sur la figure 1.

Dans le mode de réalisation représenté, comme il sera précisé par la suite, l'installation 10 est partiellement enterrée dans un sol 22, ledit sol étant défini par une surface 24. Dans la présente description, on considère que la surface 24 du sol 22 est sensiblement horizontale et orientée vers le haut.

On considère une base orthonormée (X, Y, Z) associée à l'installation 10, la direction Z représentant la verticale.

Le couloir 12 de digestion de l'installation 10 est apte à recevoir de la matière organique à digérer. Ledit couloir 12 s'étend de manière sensiblement linéaire entre une première 26 et une seconde 28 extrémités. On considère que le couloir 12 s'étend selon la direction longitudinale X. Chaque extrémité 26, 28 est matérialisée par une paroi s'étendant sensiblement dans un plan (Y, Z).

De manière générale, le couloir 12 de digestion a la forme d'un canal ouvert vers le haut.

Plus précisément, le couloir 12 de digestion comprend : une zone principale 30, une zone d'introduction 32 ; et une zone d'évacuation 34.

La zone principale 30 présente une surface 36 sensiblement cylindrique s'étendant selon la direction longitudinale X. Par « surface sensiblement cylindrique », on entend que la surface 36 est sensiblement formée de droites génératrices parallèles à X. La forme de la surface 36 dans le mode de réalisation représenté sera précisée ultérieurement.

La zone d'introduction 32 est disposée selon X entre la première extrémité 26 du couloir 12 et la zone principale 30. La zone d'évacuation 34 est disposée selon X entre la zone principale 30 et la seconde extrémité 28 du couloir 12.

Une longueur selon X de la zone principale 30 est significativement supérieure à des longueurs selon X des zones d'introduction 32 et d'évacuation 34.

Comme il sera précisé par la suite, la zone d'introduction 32 présente une largeur croissante de manière continue entre la première extrémité 26 et la zone principale 30 ; et la zone d'évacuation 34 présente une largeur décroissante de manière continue entre la zone principale 30 et la seconde extrémité 28.

Dans le mode de réalisation représenté, le couloir 12 de digestion comporte : un fond 40 ; une première et une seconde parois inclinées 42 ; une première et une seconde parois principales 44 ; une première et une seconde parois d'introduction 46 ; et une première et une seconde parois d'évacuation 48.

Dans le mode de réalisation représenté, le couloir 12 est sensiblement symétrique par rapport à un plan (X, Z).

Le fond 40 est sensiblement plan et horizontal, et s'étend selon X entre les première 26 et seconde 28 extrémités du couloir 12. Le fond 40 est délimité selon Y par un premier et un second bords latéraux 50, parallèles à X. Lesdits bords latéraux 50 sont écartés d'une première largeur 52 mesurée selon Y.

Selon un mode de réalisation, à proximité de la seconde extrémité 28, le fond 40 présente une pente faible descendant vers ladite seconde extrémité. La pente faible est par exemple de l'ordre de 1%.

Chacune des première et seconde parois inclinées 42 est sensiblement plane et s'étend vers le haut à partir, respectivement, du premier et du second bord latéral 50. Chaque paroi inclinée 42 est délimitée vers le haut par un bord d'extrémité 54, parallèle à X.

Chacune des première et seconde parois inclinées 42 est inclinée par rapport à la verticale, d'un angle α compris entre 30° et 60°, préférentiellement compris entre 40° et 50°.

Les première et seconde parois principales 44 délimitent la zone principale 30 selon X. Chacune des première et seconde parois principales 44 s'étend dans un plan (X, Z) à partir du bord d'extrémité 54, respectivement de la première et de la seconde paroi inclinée 42. Les première et seconde parois principales 44 sont écartées d'une deuxième largeur 56 mesurée selon Y.

La deuxième largeur 56 est supérieure à la première largeur 52. Préférentiellement, un ratio entre la deuxième largeur 56 et la première largeur 52 est compris entre 1,5 et 4, plus préférentiellement compris entre 2 et 3.

Les première et seconde parois d'introduction 46 délimitent la zone d'introduction 32 selon X. Chacune des première et seconde parois d'introduction 46 s'étend dans un plan vertical entre la première extrémité 26 du couloir 12 et, respectivement, la première et la seconde paroi principale 44.

Chacune des première et seconde parois d'introduction 46 est inclinée par rapport à X d'un angle β compris entre 30° et 60°, préférentiellement compris entre 35° et 55°, plus préférentiellement compris entre 40° et 50°. Ainsi, la zone d'introduction 32 présente une largeur croissante de manière continue entre la première extrémité 26 et la zone principale 30.

Chacune des première et seconde parois d'introduction 46 forme une jonction oblique 58 avec, respectivement, la première et la seconde paroi inclinée 42.

Les première et seconde parois d'évacuation 48 délimitent la zone d'évacuation 34 selon X. Chacune des première et seconde parois d'évacuation 48 s'étend dans un plan vertical entre, respectivement, la première et la seconde paroi principale 44, et la seconde extrémité 28 du couloir 12.

Chacune des première et seconde parois d'évacuation 48 est inclinée par rapport à X d'un angle γ compris entre 30° et 60°, préférentiellement compris entre 35° et 55°, plus préférentiellement compris entre 40° et 50°. Ainsi, la zone d'évacuation 34 présente une largeur décroissante de manière continue entre la zone principale 30 et la seconde extrémité 28.

Chacune des première et seconde parois d'évacuation 48 forme une jonction oblique 60 avec, respectivement, la première et la seconde paroi inclinée 42.

Chacune des premières parois d'introduction 46, principale 44 et d'évacuation 48 présente une extrémité supérieure sensiblement horizontale, lesdites extrémités supérieures formant un premier bord supérieur 62 du couloir 12. De même, chacune des secondes parois d'introduction 46, principale 44 et d'évacuation 48 présente une extrémité supérieure sensiblement horizontale, lesdites extrémités supérieures formant un second bord supérieur 62 du couloir 12. Avec les première 26 et seconde 28 extrémités, les premier et second bords supérieurs 62 confèrent une forme sensiblement octogonale à une partie supérieure du couloir 12.

De manière optionnelle, les premier et second bords supérieurs 62 sont reliés l'un à l'autre par des renforts (non représentés) tels que des sangles sous tension s'étendant selon Y. De préférence, plusieurs renforts sont répartis le long de la zone principale 30.

De manière optionnelle, le couloir 12 comporte en outre des rampes d'arrosage 63, par exemple disposées le long des parois principales 44.

Le collecteur 14 de gaz surmonte le couloir 12 de digestion, de sorte à recevoir du biogaz se dégageant de la matière organique en circulation dans ledit couloir. Plus particulièrement, le collecteur 14 forme une voûte 64 prolongeant vers le haut les bords supérieurs 62 du couloir 12, de sorte à fermer verticalement l'espace formé par ledit couloir.

La voûte 64 comporte préférentiellement une première paroi 66, formée d'une membrane imperméable au biogaz. Dans le mode de réalisation représenté, la voûte 64 comporte en outre une deuxième paroi 68 de protection, recouvrant la première paroi 66.

De préférence, le collecteur 14 comporte en outre une ou plusieurs cheminées (non représentées) d'évacuation de gaz.

Le dispositif d'introduction 16 est disposé à la première extrémité 26 du couloir 12 et est destiné à introduire dans ledit couloir de la matière organique à digérer.

Dans le mode de réalisation représenté, le dispositif d'introduction 16 comporte : un conduit d'introduction 70 ; un poussoir 72 ; et de préférence au moins un radiateur 74.

Le conduit d'introduction s'étend entre une entrée 76 et une sortie 78, ladite sortie ouvrant sur la première extrémité 26 du couloir de digestion. Plus précisément, la sortie 78 est matérialisée par une première ouverture, ménagée dans la paroi verticale formant ladite première extrémité 26. Ladite première ouverture s'étend à partir du fond 40 du couloir 12.

L'entrée 76 est matérialisée par une deuxième ouverture, ménagée dans une paroi verticale du conduit d'introduction 70. Selon la direction verticale Z, l'entrée 76 est située plus haut que la sortie 78.

Plus précisément, dans le mode de réalisation représenté, le conduit d'introduction 70 comporte : une portion de chute 80, sur laquelle débouche l'entrée 76 ; et une portion de déflexion 82, débouchant sur la sortie 78.

La portion de chute 80 s'étend verticalement, de l'entrée 76 jusqu'à la portion de déflexion 82.

Ladite portion de déflexion est configurée pour introduire la matière organique dans le couloir 12, par la sortie 78, selon un déplacement sensiblement horizontal.

A cet effet, la portion de déflexion 82 comporte une glissière 84, inclinée vers la première extrémité 26 du couloir de digestion. Dans le mode de réalisation représenté, la glissière 84 est sensiblement plane, inclinée par rapport à l'horizontale, par exemple d'un angle d'environ 45°. En variante non représentée, la glissière 84 présente une surface recourbée concave.

Le poussoir 72 est apte à se déplacer selon une course verticale dans la portion de chute 80, entre une position haute et une position basse. Dans la position haute, visible sur la figure 1, le poussoir 72 est situé au-dessus de l'entrée 76. Dans la position basse, le poussoir 72 est situé sous ladite entrée 76.

L'au moins un radiateur 74 est destiné à chauffer la matière organique introduite dans le couloir 12. L'au moins un radiateur est par exemple disposé à proximité de la glissière 84.

Selon une variante de réalisation non représentée, le dispositif d'introduction comporte : une pluralité de conduits d'introduction, alignés selon Y à la première extrémité du couloir ; et une pluralité de poussoirs, chaque poussoir équipant un conduit d'introduction. Lesdits conduits d'introduction et lesdits poussoirs sont préférentiellement analogues au conduit d'introduction 70 et au poussoir 72 décrits ci-dessus. Préférentiellement, au moins l'un desdits conduits d'introduction est équipé d'un radiateur 74 tel que décrit ci-dessus.

Le dispositif d'évacuation 18, disposé à la seconde extrémité 28 du couloir 12, est destiné à évacuer dudit couloir la matière organique digérée.

Le dispositif d'évacuation 18 comporte un conduit d'évacuation 86 et une pompe d'évacuation 88, disposée sur ledit conduit.

Le conduit d'évacuation 86 s'étend à partir d'une entrée 90, matérialisée par une troisième ouverture ménagée dans la paroi verticale formant la seconde extrémité 28 du couloir 12. De préférence, ladite troisième ouverture est légèrement surélevée par rapport au fond 40 du couloir.

De préférence, le conduit d'évacuation comporte une portion conique 92 et une portion cylindrique 94. La portion conique est adjacente à l'entrée 90 et présente un diamètre décroissant à partir de ladite entrée, jusqu'à la portion cylindrique 94. La pompe d'évacuation 88 est disposée sur ladite portion cylindrique.

De préférence, l'installation 10 comporte un local 96 adjacent à la seconde extrémité 28 du couloir 12, ledit local 96 recevant la portion conique 92, la pompe 88 et une partie de la portion cylindrique 94 du conduit d'évacuation.

Un procédé de construction de l'installation 10 va maintenant être décrit.

Une excavation 97 est tout d'abord pratiquée dans le sol 22, correspondant sensiblement à la forme du fond 40 et des parois inclinées 42 du couloir 12. Une longueur selon X de ladite excavation 97 est choisie en fonction d'une longueur souhaitée du couloir 12.

Des premiers éléments préfabriqués sont insérés dans le sol 22 pour former une structure des première 26 et seconde 28 extrémités, des parois d'introduction 46 et des parois d'évacuation 48 du couloir 12.

De même, des deuxièmes éléments préfabriqués sont disposés partiellement enterrés dans le sol 22, pour former le conduit d'introduction 70. De préférence, ledit conduit d'introduction est configuré de sorte que la deuxième ouverture, formant l'entrée 76, affleure la surface 24 du sol 22. Dans le mode de réalisation représentée, la première ouverture formant la sortie 78 est sous le niveau de ladite surface 24, de même que le fond 40.

De même, des troisièmes éléments préfabriqués sont disposés partiellement enterrés dans le sol 22, pour former le local 96.

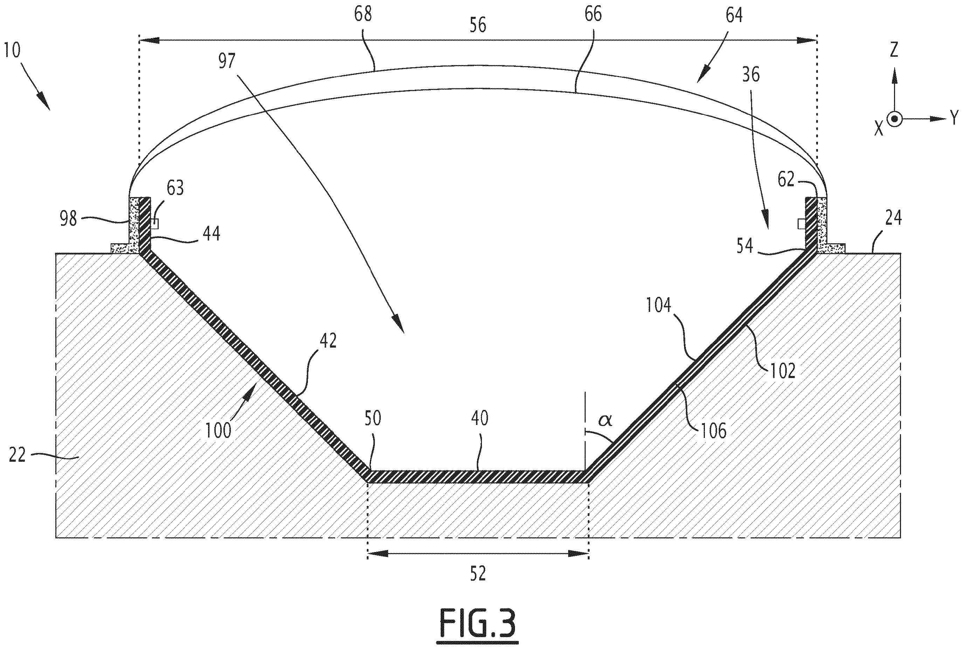

En outre, des quatrièmes éléments 98 sont disposés à la surface 24 du sol 22 pour former une structure des parois principales 44. Comme visible sur la figure 3, les quatrièmes éléments 98 ont de préférence une section en L pour stabiliser les parois principales 44 en position verticale.

Selon un mode de réalisation, les quatrièmes éléments 98 sont préfabriqués, plusieurs éléments analogues étant alignés pour atteindre la longueur souhaitée du couloir. Selon un autre mode de réalisation, les quatrièmes éléments 98 sont fabriqués sur place par coulage de béton.

De préférence, les quatrièmes éléments 98 sont solidaires d'éléments de fondation (non représentés), tels que des plots, enterrés dans le sol 22.

Les premiers, deuxièmes, troisièmes et quatrièmes 98 éléments préfabriqués sont de préférence réalisés en béton. De préférence, les premiers, deuxièmes et troisièmes éléments préfabriqués ont une taille standard ; et les quatrièmes éléments 98 préfabriqués sont adaptés à la longueur selon X souhaitée pour l'installation 10.

Les premiers et quatrièmes 98 éléments préfabriqués sont solidarisés les uns aux autres, de sorte que les extrémités 26, 28 et les bords supérieurs 62 forment une ceinture solide au couloir 12.

Ensuite, une membrane composite 100 est appliquée sur l'excavation 97 et sur les premiers et quatrièmes éléments préfabriqués, de manière à finaliser le fond 40, les parois inclinées 42, les parois principales 44, les parois d'introduction 46 et les parois d'évacuation 48 du couloir 12.

La membrane composite 100 comporte par exemple : une couche 102 de géotextile, orientée vers le sol 22 et vers les éléments préfabriqués ; une première couche 104 de matériau plastique, orientée vers l'intérieur du couloir 12 ; et un isolant 106 disposé entre lesdites couches 102 et 104. L'isolant 106 est par exemple formé de polystyrène recyclé. De préférence, la membrane composite comporte en outre une deuxième couche de matériau plastique, intercalée entre la première couche 102 de géotextile et l'isolant 106.

Ensuite, les rampes d'arrosage 63 et la voûte 64 du collecteur sont mises en place ; et les dispositifs d'introduction 16 et d'évacuation 18 sont installés aux emplacements correspondants.

Un procédé de fonctionnement de l'installation 10 de méthanisation va maintenant être décrit.

Tout d'abord, de la matière organique, telle que des déchets agricoles contenant de la paille, est déchargée dans le conduit d'introduction 70 par l'entrée 76. Lorsque la portion de chute 80 est pleine, le poussoir 72 est actionné alternativement entre la position haute et la position basse. A chaque fois que la position haute est atteinte, un nouveau déchargement de matière organique peut être effectué à l'entrée 76. Sous l'action du poussoir, le couloir 12 est progressivement rempli de matière organique.

L'au moins un radiateur 74 permet de chauffer la matière organique avant son introduction dans le couloir 12, pour accélérer le processus de digestion.

Le fonctionnement du poussoir 72 et de l'au moins un radiateur 74 est par exemple géré par un programme mémorisé dans le module électronique 20 de commande.

La forme à largeur croissante de la zone d'introduction 32 favorise un chargement homogène du couloir 12 en matière organique, en évitant la formation de zones mortes.

En raison des propriétés physiques évoquées ci-dessus, l'introduction continue de matière organique conduit ladite matière à se déplacer de manière cohérente le long du couloir 12, selon la direction X. De préférence, ladite matière organique est arrosée tout au long de son parcours, au moyen des rampes d'arrosage 63, injectant de l'eau ou des jus de fermentation dans le couloir 12. Le fonctionnement des rampes d'arrosage est préférentiellement géré par le programme du module électronique 20.

La progression de la matière organique le long du couloir 12 de digestion, dans un environnement humide et tiède, favorise la décomposition anaérobie de ladite matière organique. Il s'ensuit une production de biogaz qui se dégage de la matière solide et est récupéré dans la voûte 64 du collecteur 14. De préférence, le gaz est ensuite évacué par la ou les cheminées.

A titre indicatif, le temps de parcours de la matière organique, entre les première 26 et seconde 28 extrémités du couloir 12, est compris entre 40 et 60 jours. Le temps de parcours dépend notamment de la longueur selon X choisie pour le couloir 12.

La forme à largeur décroissante de la zone d'évacuation 34 du couloir 12 favorise le déplacement de la matière organique vers l'entrée 90 du conduit 86 d'évacuation.

Lorsque la matière organique digérée arrive à la seconde extrémité 28 du couloir 12, elle est aspirée dans le conduit 86 d'évacuation par la pompe 88. Le fonctionnement de ladite pompe est préférentiellement géré par le programme du module électronique 20.

De préférence, la matière organique digérée est évacuée par le conduit 86 vers un séparateur de phases et/ou un bâtiment de stockage de digestat (non représentés). La phase liquide est éventuellement recyclée par réintroduction dans le couloir 12 au moyen des rampes d'arrosage 63.

L'installation 10 de méthanisation selon l'invention permet d'obtenir une circulation efficace de la matière organique en digestion. Le débit de matière traitée est ainsi amélioré. A titre d'exemple, l'installation 10 de méthanisation décrite ci-dessus permet d'obtenir des débits de 1000 m³ à 3000 m³ par jour de biogaz, pour une première 52 et une deuxième 56 largeurs respectivement de l'ordre de 4 m et de 12 m pour le couloir 12.

## Revendications

1. Installation (10) de méthanisation, comprenant :
- un couloir (12) de digestion, apte à recevoir de la matière organique, ledit couloir s'étendant de manière sensiblement linéaire selon une direction longitudinale horizontale (X), entre une première (26) et une seconde (28) extrémités ;
- un collecteur (14) de gaz, surmontant le couloir de digestion ; et
- un dispositif d'introduction (16) et un dispositif d'évacuation (18) de matière organique, disposés respectivement à la première et à la seconde extrémités du couloir de digestion ;
le couloir de digestion comprenant une zone principale (30), ladite zone principale présentant une surface sensiblement cylindrique s'étendant selon la direction longitudinale ;
l'installation étant **caractérisée en ce que** le couloir de digestion comporte en outre :
- une zone d'introduction (32), présentant une largeur croissante de manière continue entre la première extrémité et la zone principale ; et
- une zone d'évacuation (34), présentant une largeur décroissante de manière continue entre la zone principale et la seconde extrémité.

2. Installation de méthanisation selon la revendication 1, dans laquelle, selon une direction transversale (Y), perpendiculaire à la direction longitudinale, au moins l'une de la zone d'introduction et de la zone d'évacuation est délimitée par deux premières parois (46, 48) sensiblement planes.

3. Installation de méthanisation selon la revendication 2, dans laquelle chacune des deux premières parois (46, 48) est sensiblement verticale.

4. Installation de méthanisation selon la revendication 2 ou 3, dans laquelle chacune des premières parois (46, 48) forme un premier angle (β, y) compris entre 30° et 60°, préférentiellement compris entre 35° et 55°, plus préférentiellement compris entre 40° et 50°, avec la direction longitudinale.

5. Installation de méthanisation selon l'une des revendications précédentes, dans laquelle le couloir (12) de digestion comporte deux deuxièmes parois (42) orientées vers le haut, chacune desdites deuxièmes parois étant inclinée par rapport à la verticale, d'un deuxième angle (α) compris entre 30° et 60°, préférentiellement compris entre 40° et 50°.

6. Installation de méthanisation selon la revendication 5, dans laquelle le couloir (12) de digestion comporte en outre un fond (40) sensiblement horizontal, reliant les deux deuxièmes parois (42).

7. Installation de méthanisation selon la revendication 5 ou 6, dans laquelle la zone principale (30) du couloir de digestion comporte deux troisièmes parois (44) sensiblement verticales, chacune desdites troisièmes parois prolongeant vers le haut l'une des deuxièmes parois (42).

8. Installation de méthanisation selon l'une des revendications précédentes, dans laquelle le dispositif d'introduction (16) comporte : un conduit d'introduction (70), ouvrant sur la première extrémité (26) du couloir de digestion ; et un poussoir (72), apte à pousser la matière organique vers ladite première extrémité.

9. Installation de méthanisation selon la revendication 8, dans laquelle le conduit d'introduction (70) comporte : une portion de chute (80), s'étendant verticalement ; et une portion de déflexion (82), configurée pour introduire la matière organique dans le couloir de digestion selon un déplacement sensiblement horizontal ; le poussoir étant apte à se déplacer selon une course verticale dans la portion de chute.

10. Procédé de construction d'une installation selon l'une des revendications 5 à 7, comprenant les étapes suivantes : réalisation d'une excavation (97) dans un sol (22) ; et application d'une membrane (100) sur ladite excavation, de sorte à former les deuxièmes parois (42).
